# EUROPEAN PATENT APPLICATION

(11) **EP 4 636 094 A1**
(43) Date of publication of application: **22.10.2025**
(21) Application number: 22968271.1
(22) Date of filing: 16.12.2022
(51) Int. Cl.: C12Q 1/68

(54) **NANOPORE SEQUENCING METHOD AND KIT**

(71) Applicant: BGI Shenzhen, Shenzhen, Guangdong 518083 (CN); BGI Shenzhen Co., Ltd, Shenzhen, Guangdong 518083 (CN)
(72) Inventor: CHEN, Junyi, Shenzhen, Guangdong 518083 (CN); ZENG, Tao, Shenzhen, Guangdong 518083 (CN); JI, Zhouxiang, Shenzhen, Guangdong 518083 (CN); LI, Yuxiang, Shenzhen, Guangdong 518083 (CN); DONG, Yuliang, Shenzhen, Guangdong 518083 (CN); ZHANG, Wenwei, Shenzhen, Guangdong 518083 (CN); XU, Xun, Shenzhen, Guangdong 518083 (CN)
(74) Representative: Schwarz & Partner Patentanwälte GmbH
(86) International application number: PCT/CN2022/139739
(87) International publication number: WO 2024/124567

(57) **Abstract**

Provided are a nanopore sequencing method and a kit. The method comprises: 1) providing a sequencing library, comprising target polynucleotide double strands, wherein the target polynucleotide double strands comprise a first strand and a second strand, and a 5' end of the first strand and a 3' end ofthe second strand comprise non-paired first single-stranded regions, which comprise a guide sequence and a second primer pairing region, respectively; 2) incubating a first primer and the sequencing library to for a sequencing complex, wherein the first primer has a 3' free end and is attached, by means of a 5' end, to a membrane embedded with a nanopore; 3) making a polymerase with strand displacement activity extend, by using the second strand as a template, the first primer, wherein under the action of an electric field force, the guide sequence is captured by the nanopore on the membrane and passes through the nanopore, and as the extension proceeds, the first strand is obtained by displacement and paases through the nanopore; and 4) detecting an electric signal change generated when the first strand passes through the nanopore in the extension process, and determining sequence information of the first strand of the target polynucleotide double strands.

## Description

### FIELD

The present disclosure relates to the technical field of biotechnology, in particular to a method and a kit for nanopore sequencing.

### BACKGROUND

Nanopore sequencing technology is a single-molecule detection technique that offers advantages such as rapid sequencing speed, long read lengths, direct sequencing, high throughput, low cost, compact size, and portability. During nanopore sequencing, a single nanopore is embedded in an insulating and impermeable membrane, forming a stable ion current channel. Under a voltage, single-stranded nucleic acid molecule passes through the nanopore, causing a reduction in the ion current across the nanopore. Due to differences in molecular structure and size among the bases of the single-stranded nucleic acid molecule, the current across the nanopore exhibits changes corresponding to the respective base sequences. By decoding the current signal change using algorithms, the sequence of the single-stranded nucleic acid passing through the nanopore may be read in real time. However, a single-stranded nucleic acid without control passes through the nanopore too fast to decode the generated electrical signal. Additionally, during normal sequencing, a double-stranded nucleic acid must first be converted into a single-stranded nucleic acid, before passing through the nanopore and being sequenced.

In the related technology, various strategies have been developed to control the conversion of a double-stranded nucleic acid to be tested into a single-stranded nucleic acid at a relatively stable speed, thereby enabling the single-stranded nucleic acid to pass through the nanopore at the relatively stable speed for sequencing.

Patent disclosures CN105209634A and CN106460061A disclose the following route of sequencing technology: (i) using a Y-shaped sequencing adapter complex, wherein the top strand of the adapter consists of "a guide sequence + a helicase binding site + a spacer + a bottom strand complementary sequence", the bottom strand of the adapter consists of "a top strand complementary sequence + a tether complementary sequence", such that the top and bottom strands forms the sequencing adapter after annealing, and a helicase may bind to "the helicase binding site" on the top strand of the adapter and be stopped by the spacer sequence; (ii) ligating the nucleic acid to be tested with the Y-shaped sequencing adapter complex to obtain a sequencing library; (iii) adding the sequencing library, the tether and sequencing buffer into sequencing flow cells embedded with nanopores, wherein the tether enables the sequencing library bind to the high-molecular polymer membrane embedded with nanopore proteins; (iv) enabling the helicase move and pass through the spacer under the electric field force when the sequencing library was captured by the nanopores, thereby unwinding the double stranded nucleic acids for sequencing, wherein the helicase will be unable to unwind the double stranded nucleic acids due to the presence of the spacer if the sequencing library is not captured by the nanopores. The disadvantages of this route are: (a) the Y-shaped sequencing adapter complex used, which is a helicase-nucleic acid complex, has a complicated construction process and a low yield; (b) the sequencing library constructed with the Y-shaped sequencing adapter complex, for which the biochemical activity of the helicase and the integrity of the nucleic acid are needed at the same time, requires strict preservation conditions, which makes it difficult to be preserved for a long period of time, and the sequencing for the library must be carried out as soon as possible.

U.S. Patent US10480027B2 discloses the following route of sequencing technology: (i) providing a polymerase-nucleic acid complex and the necessary components for nucleic acid synthesis, to synthesize a nascent strand, wherein the polymerase-nucleic acid complex includes a polymerase with strand-displacement activity and a circular nucleic acid template; (ii) determining the sequence of the nascent strand by measuring the current changes generated as the nascent strand passes through the nanopore over the time. Patent disclosure CN113366120A also describes a similar route. The disadvantages of this route is: the synthesis of the nascent strand continuously proceeds and cannot be controlled until it is captured by the nanopore, however, the nascent strand is prone to form complicated secondary structures, which makes it difficult to be captured by the nanopore to complete the sequencing.

Therefore, there is a need for improved nanopore sequencing technology in this field.

### SUMMARY

The present disclosure aims to solve or improve, at least in part, one or more of the technical problems in the nanopore sequencing technology as follows: the Y-shaped sequencing adapter complex has a complicated construction process and a low yield, and requires strict preservation conditions, which makes it difficult to be preserved for a long period of time; the nascent strand synthesized by polymerase is prone to form secondary structures before passing through the nanopore; the sequencing system is highly complicated.

Therefore, in a first aspect, embodiments of the present disclosure propose a method for nanopore sequencing, including:
i) providing a sequencing library, wherein the sequencing library includes a target double stranded polynucleotide, the target double stranded polynucleotide includes a first strand and a second strand, a 5' end of the first strand and a 3' end of the second strand form an first unpaired single-stranded region, the first unpaired single-stranded region is provided with a guide sequence at the 5' end of the first strand, the first unpaired single-stranded region is provided with a second primer pairing region at the 3' end of the second strand;
ii) incubating the sequencing library with a first primer to obtain a sequencing complex, wherein the first primer binds to the second primer pairing region based on a base complementary pairing principle, the first primer has a free 3' end and has a 5' end capable of attaching the complex to a membrane with a nanopore embedded;
iii) extending the first primer with the second strand serving as a template mediated by a first polymerase having a strand displacement activity, wherein, under an electric field force, the guide sequence is captured by the nanopore embedded in the membrane and passes through the nanopore, and the first strand of the target double stranded polynucleotide is displaced out and passes through the nanopore as the first primer extends; and
iv) detecting the electrical signal changes generated as the first strand passes through the nanopore during extension, to determine sequence information for the first strand of the target double stranded polynucleotide.

In a preferred embodiment, in i), the 3' end of the first strand and the 5' end of the second strand form a paired region under completely complementarily pairing.

In a preferred embodiment, in i), in the target double stranded polynucleotide, a 3' end of the first strand and a 5' end of the second strand form a second unpaired single-stranded region, the second unpaired single-stranded region is provided with a first primer pairing region at the 3' end of the first strand.

In a preferred embodiment, in ii), incubating the sequencing library with the first primer, a second primer to obtain the sequencing complex, wherein the first primer binds to the second primer pairing region based on the base complementary pairing principle, the second primer binds to the first primer pairing region based on the base complementary pairing principle, and the 5' end(s) of the first primer and/or the second primer is(are) capable of attaching the complex to the membrane with the nanopore embedded.

In a preferred embodiment, the method further includes: v) extending the second primer with the first strand serving as a template mediated by a second polymerase such that the first strand exits the nanopore embedded in the membrane in a direction inverse to the electric field, and detecting the electrical signal changes generated as the first strand exits the nanopore, to determine the sequence information for the first strand of the target double stranded polynucleotide again.

In a preferred embodiment, the second strand is provided with a guide sequence at the 5' end.

In a preferred embodiment, in i), in the target double stranded polynucleotide, a 3' end of the first strand and a 5' end of the second strand are connected, such that the target double stranded polynucleotide is provided with a hairpin structure at the connection.

In a preferred embodiment, the method further includes: v) continuing extending with the first strand serving as a template mediated by the first polymerase after extending to the hairpin structure, such that the first strand exits the nanopore embedded in the membrane in a direction inverse to the electric field, and detecting the electrical signal changes generated as the first strand exits the nanopore, to determine the sequence information for the first strand of the target double stranded polynucleotide again.

In some embodiments, the polymerase having the strand displacement activity is a salt-tolerant polymerase.

In some embodiments, the polymerase having the strand displacement activity is DNA polymerase or RNA polymerase, such as a Bst DNA polymerase, a SD DNA polymerase, a phi29 DNA polymerase, a Bsu Large Fragment DNA polymerase, a Klenow Fragment DNA polymerase, a T3 RNA polymerase, a T7 RNA polymerase, a SP6 RNA polymerase, an *E.coli* RNA polymerase, or any combination thereof; or a modified polymerase having the strand displacement activity, which is a polymerase without strand displacement activity originally, such as a modified T4 DNA polymerase, a modified T7 DNA polymerase, a modified DNA polymerase I, or any combination thereof.

In an embodiment, the target double stranded polynucleotide is prepared by linking a Y-adapter to a double stranded polynucleotide, two strands of the Y-adapter include the guide sequence and a primer pairing region respectively, such that the target double stranded polynucleotide, obtained by linking the Y-adapter to the double stranded polynucleotide, is provided with the guide sequence at the 5' end of the first strand and the second primer pairing region at the 3' end of the second strand.

In an embodiment, the primer is single stranded nucleic acid having less than 80, for example, less than 70, 60 or 50 nucleotides in length.

In an embodiment, the 5' end of the primer is attached with a hydrophobic molecule, preferably selected from any one or more of the following: a lipid, fatty acid, a sterol, a carbon nanotube, a peptide, a protein and/or an amino acid, such as cholesterol, palmitate or tocopherol.

In an embodiment, the guide sequence includes a polynucleotide or a plurality of iSpC3s. Preferably, the polynucleotide is a homopolymeric polynucleotide.

In an embodiment, the guide sequence is 10-50 nucleotides or 10-50 iSpC3s, such as 20, 30 or 40 nucleotides or iSpC3s, in length.

In some embodiments, the nanopore is a transmembrane protein pore or a solid-state pore.

In some embodiments, the transmembrane protein pore is selected from hemolysin, MspA, MspB, MspC, MspD, FraC, ClyA, PA63, CsgG, CsgD, XcpQ, SP1, phi29 connector protein, InvG, GspD, or any combination thereof.

In some embodiments, the nanopore is further attached with an additional polypeptide selected from a tag, an enzyme cleavage site, a signaling peptide or a guide peptide, a detectable marker or any combination thereof.

In some embodiments, the membrane is an amphiphilic membrane (such as a phospholipid bilayer), a high-molecular polymer membrane (such as a diblock copolymer and a triblock copolymer) or any combination thereof.

In some embodiments, a 3' end of the guide sequence is linked to an inhibitory segment that inhibits the strand displacement activity of the polymerase.

In some embodiments, the inhibitory segment is a GC-rich motif, an artificially modified nucleotide, other inhibitory molecule or any combination thereof.

In some embodiments, the artificially modified nucleotide is a locked nucleic acid (LNA), a peptide nucleic acid (PNA), a bridged nucleic acid (BNA), or any combination thereof.

In some embodiments, the artificially modified nucleotide is the LNA, whose number is 2-10, preferably 4-8.

In some embodiments, the method is carried out in a reaction buffer or sequencing buffer as follows: a dihydrogen phosphate-hydrogen phosphate buffer system, a carbonic acid-sodium bicarbonate buffer system, a Tris-HCl buffer system, a HEPES buffer system, a MOPS buffer system or any combination thereof.

In some embodiments, the reaction buffer or sequencing buffer contains NTP, dNTP, ddNTP, or any combination thereof.

In some embodiments, the reaction buffer or sequencing buffer contains K+ , Na+ or any combination thereof.

In some embodiments, the reaction buffer or sequencing buffer contains Mg2+, Mo2+, Cu2+, Fe2+, Zn2+, Ca2+, Pb2+, Cd2+, or any combination thereof.

In some embodiments, the reaction buffer or sequencing buffer contains an additive or an auxiliary reagent that enhances a polymerase extension reaction, such as dimethyl sulfoxide (DMSO), glycerol, formamide, bovine serum albumin (BSA), ammonium sulfate, polyethylene glycol (PEG), gelatin, non-ionic detergent (such as Tween 20, Trtion X-100), N,N,N-trimethylglycine (betaine), single-stranded nucleic acid-binding protein or any combination thereof.

In some embodiments, a voltage is applied to both sides of the membrane to form the electric field force. In some embodiments, the voltage is a voltage of 10 or more mV, preferably 50 mV-250 mV.

In a second aspect, embodiments of the present disclosure propose a kit including:
a Y-adapter for linking to a double stranded polynucleotide, wherein two strands of the Y-adapter include a guide sequence and a primer pairing region respectively, such that a target double stranded polynucleotide, obtained by linking the Y-adapter to the double stranded polynucleotide, is provided with the guide sequence at the 5' end of a first strand and the primer pairing region at the 3' end of a second strand;
a nanopore;
a membrane;
one or more primer, wherein the primer is complementary to the primer pairing region, and the primer has an attaching segment at the 5' end for attaching to the membrane;
one or more polymerase having a strand displacement activity;
a buffer system for a polymerization reaction.

In some embodiments, the nanopore is embedded in the membrane.

In some embodiments, a 3' end of the guide sequence is attached with an inhibitory segment that inhibits the strand displacement activity of the polymerase.

In some embodiments, the inhibitory segment is a GC-rich motif or an artificially modified nucleotide.

In some embodiments, the artificially modified nucleotide is LNA, PNA, BNA, or any combination thereof.

In some embodiments, the artificially modified nucleotide is 2-10 LNAs, more preferably the artificially modified nucleotide is 4-8 LNAs .

In some embodiments, the primer is a single stranded nucleic acid having less than 80, such as less than 70, 60 or 50 nucleotides in length.

In some embodiments, a 5' end of the primer is attached with a hydrophobic molecule, and the hydrophobic molecule is preferably selected from any one or more of the following: a lipid, fatty acid, a sterol, a carbon nanotube, a peptide, a protein and/or an amino acid, such as cholesterol, palmitate or tocopherols.

In some embodiments, the guide sequence includes a polynucleotide or iSpC3.

In some embodiments, the guide sequence is 10-50 nucleotides or 10-50 iSpC3s, such as 20, 30, or 40, in length.

In some embodiments, the polymerase having the strand displacement activity is DNA polymerase or RNA polymerase.

In some embodiments, the polymerase having the strand displacement activity is a salt-tolerant polymerase.

In some embodiments, the polymerase is a modified polymerase having the strand displacement activity, which is a polymerase without strand displacement activity originally.

In some embodiments, the polymerase having the strand displacement activity is DNA polymerase or RNA polymerase, such as a Bst DNA polymerase, a SD DNA polymerase, a phi29 DNA polymerase, a Bsu Large Fragment DNA polymerase, a Klenow Fragment DNA polymerase, a T3 RNA polymerase, a T7 RNA polymerase, a SP6 RNA polymerase, an *E.coli* RNA polymerase, or any combination thereof; or the polymerase is a modified polymerase having the strand displacement activity, which is a polymerase without strand displacement activity originally, such as a modified T4 DNA polymerase, a modified T7 DNA polymerase, a modified DNA polymerase I, or any combination thereof.

In some embodiments, the nanopore is a transmembrane protein pore or a solid-state pore.

In some embodiments, the transmembrane protein pore is selected from hemolysin, MspA, MspB, MspC, MspD, FraC, ClyA, PA63, CsgG, CsgD, XcpQ, SP1, phi29 connector protein, InvG, GspD, or any combination thereof.

In some embodiments, the nanopore is further attached with an additional polypeptide selected from a tag, an enzyme cleavage site, a signaling peptide or a guide peptide, a detectable marker or any combination thereof.

In some embodiments, the membrane is an amphiphilic membrane, a high-molecular polymer membrane or any combination thereof.

In some embodiments, the membrane is a phospholipid bilayer, a diblock copolymer or a triblock copolymer.

In some embodiments, the buffer system for the polymerization reaction is a dihydrogen phosphate-hydrogen phosphate buffer system, a carbonic acid-sodium bicarbonate buffer system, a Tris-HCl buffer system, a HEPES buffer system, a MOPS buffer system or any combination thereof.

In some embodiments, the buffer system for the polymerization reaction contains NTP, dNTP, ddNTP, or any combination thereof.

In some embodiments, the buffer system for the polymerization reaction contains an additive or an auxiliary reagent that enhances a polymerase extension reaction, such as dimethyl sulfoxide (DMSO), glycerol, formamide, bovine serum albumin (BSA), ammonium sulfate, polyethylene glycol (PEG), gelatin, non-ionic detergent (such as Tween 20, Trtion X-100), N,N,N-trimethylglycine (betaine), single-stranded nucleic acid-binding protein or any combination thereof.

In a third aspect, embodiments of the present disclosure propose a use of the kit according to the second aspect of the present disclosure in high throughput sequencing, preferably, wherein the high throughput sequencing is nanopore sequencing.

The advantages of the present disclosure lie in one or more of the following aspects: The sequencing library and sequencing adapters of the present disclosure are characterized by their simplicity in structure, the simple and convenient construction process of the sequencing library, and the capability for long-term storage of the constructed sequencing library. The method of the present disclosure unwinds the double stranded nucleic acids and synthesizes nascent strands with a polymerase with strand-displacing activity at a relatively constant rate, thereby ensuring the strand to be sequenced to pass through the nanopore in a relatively stable speed. The method of the present disclosure ensures that DNA synthesis and the generation of nascent strands continue only after the sequencing library is captured by the nanopore. This effectively solves the problem of "the synthesis of nascent strand continuously proceeds until it is captured by the nanopore, however, the nascent strand is prone to form complicated secondary structures, which makes it difficult to be captured by the nanopore to complete the sequencing".

### BRIEF DESCRIPTION OF THE DRAWINGS

The drawings required for describing the specific embodiments or the prior art will be briefly introduced below, to illustrate the specific embodiments of the present invention or the technical solutions in the prior art more clearly. It is obvious that the drawings in the following description are some embodiments of the present invention. For those ordinary skilled in the art, other drawings can be obtained based on these drawings without exerting inventive efforts.
FIG. 1 shows a schematic diagram of a method for nanopore sequencing according to an embodiment of the present disclosure.
FIG. 2 shows a schematic diagram of a method for nanopore sequencing according to another embodiment of the present disclosure.
FIG. 3 shows a schematic diagram of a method for nanopore sequencing according to a further embodiment of the present disclosure.
FIG. 4 shows a schematic diagram of the experiments of Example 1 and Example 2, wherein (a) shows that a polynucleotide strand A and strand B form a Y-shaped double-stranded nucleic acid structure after annealing, and (b) shows that the strand A contains 2 LNAs, 4 LNAs, and 8 LNAs (denoted by the bolded black line).
FIG. 5 shows an electrophoretogram of annealing products of the Y-shaped double-stranded nucleic acid in Examples 1 and 2. Lanes 1-4 are the annealing products of SEQ.1+SEQ.2 (Y-0LNA), SEQ.4+SEQ.2 (Y-2LNA), SEQ.5+SEQ.2 (Y-4LNA), and SEQ.6+SEQ.2 (Y-8LNA), respectively, with arrows indicating the Y-shaped double-stranded nucleic acids; lanes 5-9 are the annealing products of SEQ.1, SEQ.4, SEQ.5, SEQ.6, and SEQ.2, respectively; lane 10 is DNA Marker.
FIG. 6 shows screening results of polymerases with strand displacement activity and salt tolerance in Example 1.
FIG. 7 shows detecting results of the ability of modified nucleotides in a non-template strand for inhibiting strand displacement polymerization activity of the polymerase, in Example 2. y-0LNA has 0 LNA in the non-template strand, y-2LNA has 2 LNAs in the non-template strand, y-4LNA has 4 LNAs in the non-template strand, and y-8LNA has 8 LNAs in the non-template strand.
FIG. 8 shows a schematic diagram of the sequencing library construction in Example 3, (a) shows a schematic diagram of a sequencing adapter and (b) shows a schematic diagram of a sequencing library.
FIG. 9 shows a gel electrophoresis image of the sequencing library in Example 3.
FIG. 10 shows a schematic diagram of a sequencing complex in Example 4.
FIG. 11 shows a current signal image of a typical target polynucleotide to be tested for nanopore sequencing in Example 4.
FIG. 12 shows a specific structure of iSp18 used in embodiments.
FIG. 13 shows a specific structure of iSpC3 used in embodiments.

### DETAILED DESCRIPTION

In order to make the above-mentioned and other features and advantages of the present disclosure more apparent, the present disclosure is further described below with reference to the drawings. It should be understood that the specific embodiments provided herein are only illustrative, for the purpose of explaining the present disclosure to those skilled in the art, and are not limiting. For those ordinary skilled in the art, the specific meanings of the terms in the present disclosure can be understood based on the specific context, unless explicitly defined otherwise. Furthermore, without conflicting with each other, those skilled in the art may combine and integrate different embodiments or examples described in this specification, as well as features of different embodiments or examples.

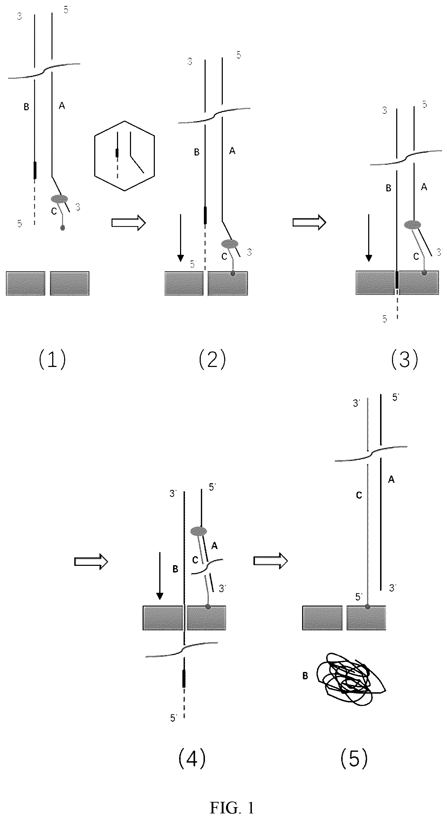

FIG. 1 shows an exemplary process of the method for nanopore sequencing according to an embodiment of the present disclosure. A Y-shaped double-stranded sequencing adapter (a hexagon-circled structure in the figure) is formed by the top and bottom strands after annealing, and is further linked to one end of the target double-stranded polynucleotide, such that the sequencing library is constructed. The constructed sequencing library may be preserved for long-term storage following conventional methods applicable to double-stranded polynucleotides. The constructed sequencing library consists of a strand A and a strand B, wherein the strand B is a strand to be sequenced and the strand A is a complementary strand of the strand to be sequenced, a 3' end of the strand A is capable of complementarily pairing with a primer strand C, a 5' end of the strand B is provided with a guide sequence (indicated by a black dotted line in the figure, referring to the patent disclosure CN 103733063A), a 3' end of the guide sequence is linked to an inhibitory segment (indicated by the bold black line in the figure) inhibiting the strand-displacement polymerization activity of a polymerase. The specific form of the inhibitory segment is not limited, as long as the inhibitory molecule is capable of inhibiting the strand-displacement polymerization activity of the polymerase. For example, the inhibitory segment may be a GC-rich motif with stronger binding ability, an artificially modified nucleotide (e.g., LNA, PNA, BNA), or other inhibitory molecules. Both the guide sequence and the inhibitory segment are positioned on the strand B. The whole sequencing process is as follows: (i) Mixing and incubating the sequencing library (strand A and strand B), the primer (strand C), and the polymerase with strand-displacement activity (represented by an ellipse in the figure), wherein the strand C binds to the 3' end of the strand A, and the polymerase with strand-displacement activity binds to the target polynucleotide, thus forming the sequencing complex. (ii) Initiating a strand displacing polymerization reaction with the strand A serving as a template and the strand C serving as the primer, mediated by the polymerase with strand displacement activity utilizing the necessary components in the sequencing buffer, such as pH buffer system, dNTP, Mg2+, etc. Wherein, the strand C is extended with the strand A serving as the template, as the strand B is displaced out, until the strand displacement polymerization activity of the polymerase is inhibited by the inhibitory segment. Since the 5' end of the strand C is attached with hydrophobic molecules such as cholesterol (represented as circles in the figure), the sequencing complex attached on the membrane material in the vicinity of the nanopore with the action of the hydrophobic molecules such as cholesterol (the membrane is represented as squares, and the center channel indicates the nanopore in the figure, with reference to patent disclosure CN 103733063A). The 5' end of the primer may further be attached with other hydrophobic molecules, such as lipids, fatty acids, sterols, carbon nanotubes, peptides, proteins and/or amino acids such as palmitate, tocopherols. The primer may further contain a variety of natural or non-natural modified nucleotides. With a sequencing voltage being applied, the guide sequence at the 5' end of the strand to be sequenced (the strand B) is captured by and passes through the nanopore under the electric field force (indicated by a downward arrow in the figure). (iii) Enabling the inhibitory segment to pass through the nanopore under the electric field force. As the strand B is displaced out, the strand C is extended in a stable speed with the strand A serving as the template, mediated by the polymerase with strand displacement activity. (iv) Enabling sequencing in a stable speed, as the strand B is displaced out and passes through the nanopore in a stable speed. (v) Completing the sequencing, wherein the strand C extends to the end of the strand A thereby forming a new double strand, and the displaced Strand B completely passes through the nanopore.

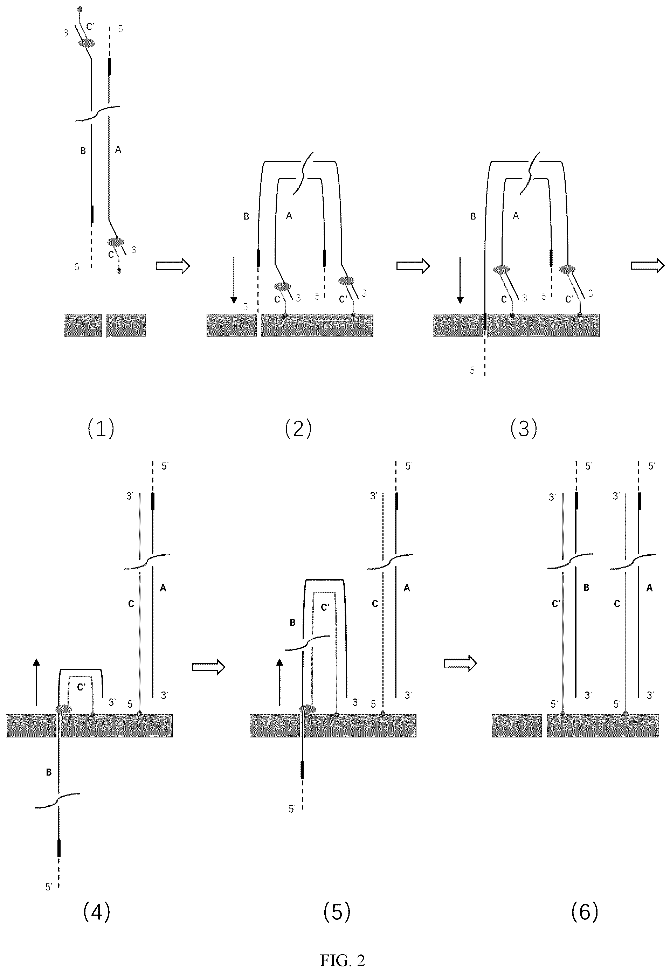

FIG. 2 shows another exemplary process of the method for nanopore sequencing according to an embodiment of the present disclosure. A Y-shaped double-stranded sequencing adapter is formed by the top and bottom strands after annealing, further, the Y-shaped double-stranded sequencing adapter 1 is linked to one end of the target double-stranded polynucleotide, and the Y-shaped double-stranded sequencing adapter 2 is linked to the other end of the target double-stranded polynucleotide such that the sequencing library is constructed. The Y adapter 1 and Y adapter 2 may be the same or different, and the specific sequences of which are not limited. The constructed sequencing library may be preserved for long-term storage following conventional methods applicable to double-stranded polynucleotides. The constructed sequencing library consists of a strand A and a strand B, wherein the strand B is a strand to be sequenced and the strand A is a complementary strand of the strand to be sequenced. A 3' end of the strand A is capable of complementarily pairing with a primer strand C, a 5' end of the strand B is provided with a guide sequence 1 (indicated by a black dotted line in the figure), a 3' end of the guide sequence is linked to an inhibitory segment 1 (indicated by the bold black line in the figure) inhibiting the strand-displacement polymerization activity of a polymerase. The specific form of the inhibitory segment 1 is not limited, as long as the inhibitory molecule is capable of inhibiting the strand-displacement polymerization activity of the polymerase. For example, the inhibitory segment 1 may be a GC-rich motif with stronger binding ability, an artificially modified nucleotide (e.g., LNA, PNA, BNA), or other inhibitory molecules. Both the guide sequence 1 and the inhibitory segment 1 are positioned on the strand A. Similarly, a 3' end of the strand B is capable of complementarily pairing with a primer strand C', a 5' end of the strand B is provided with a guide sequence 2 (indicated by a black dotted line in the figure), a 3' end of the guide sequence is linked to an inhibitory segment 2 (indicated by the bold black line in the figure) inhibiting the strand-displacement polymerization activity of a polymerase. The specific form of the inhibitory segment 2 is not limited, as long as the inhibitory molecule is capable of inhibiting the strand-displacement polymerization activity of the polymerase. For example, the inhibitory segment 2 may be a GC-rich motif with stronger binding ability, an artificially modified nucleotide (e.g., LNA, PNA, BNA), or other inhibitory molecules. Both the guide sequence 2 and the inhibitory segment 2 are positioned on the strand B. The whole sequencing process is as follows: (i) Mixing and incubating the sequencing library (strand A and strand B), the primers (strand C and strand C'), and the polymerase with strand-displacement activity (represented by an ellipse in the figure), wherein the strand C is complementarily paired with the 3' end of the strand A, and the strand C' is complementarily paired with the 3' end of the strand B, and the polymerase with strand-displacement activity binds to the target polynucleotide, thus forming the sequencing complex. (ii) Initiating a strand displacing polymerization reaction with the strand A serving as a template and the strand C serving as the primer, mediated by the polymerase with strand displacement activity at the one end of the target double stranded polynucleotides, utilizing the necessary components in the sequencing buffer, such as pH buffer system, dNTP, Mg2+, etc. Wherein, the strand C is extended with the strand A serving as the template, as the strand B is displaced out, until the strand displacement polymerization activity of the polymerase is inhibited by the inhibitory segment 2. Similarly, initiating a strand displacing polymerization reaction with the strand B serving as a template and the strand C' serving as the primer, mediated by the polymerase with strand displacement activity at the other end of the target double stranded polynucleotides, utilizing the necessary components in the sequencing buffer, such as pH buffer system, dNTP, Mg2+, etc. Wherein, the strand C is extended with the strand B serving as the template, as the strand A is displaced out, until the strand displacement polymerization activity of the polymerase is inhibited by the inhibitory segment 1. Since the 5' ends of the strand C and the strand C' are attached with hydrophobic molecules such as cholesterol (represented as circles in the figure), the sequencing complex attached on the membrane material in the vicinity of the nanopore with the action of the hydrophobic molecules such as cholesterol (the membrane is represented as squares, and the center channel indicates the nanopore in the figure). With a sequencing voltage being applied, the guide sequence 2 at the 5' end of the strand to be sequenced (the strand B) is captured by and passes through the nanopore under the electric field force (indicated by a downward arrow in the figure). (iii) Enabling the inhibitory segment 2 of the strand B to pass through the nanopore under the electric field force. As the strand B is displaced out, the strand C is extended in a stable speed with the strand A serving as the template, mediated by the polymerase with strand displacement activity. (iv) Enabling sequencing in a stable speed, as the strand B is displaced out and passes through the nanopore in a stable speed. (v) Forming a new double strand as the strand C extends to the end of the strand A, and when the displaced strand B passes through the nanopore and encounters the polymerase at the other end, initiating polymerization reaction with the strand B serving as a template to extend the strand C', utilizing the necessary components in the sequencing buffer, such as pH buffer system, dNTP, Mg2+, etc. (vi) Enabling the strand B to exit from the nanopore in a direction inverse to the electric field, in a relatively stable speed, as the strand C' extends (indicated by an upward arrow in the figure). (vii) Forming a new double strand as the strand C' extends to the end of the strand B, when the strand B exits completely from the nanopore. The same Strand B is sequenced twice during the whole process.

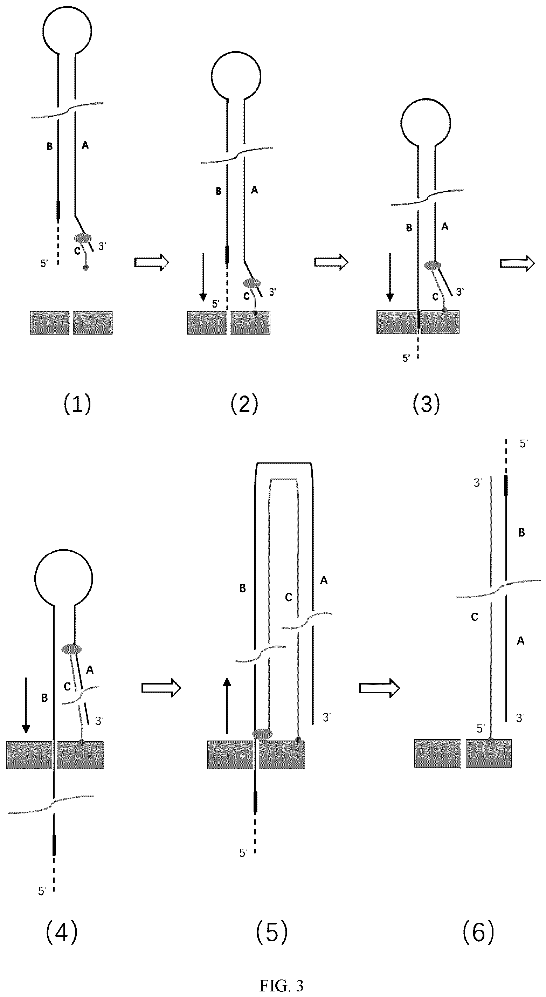

FIG. 3 shows another exemplary process of the method for nanopore sequencing according to an embodiment of the present disclosure. A Y-shaped double-stranded sequencing adapter is formed by the top and bottom strands after annealing, a hairpin-shaped adapter is formed by a single strand after annealing, further, the Y-shaped double-stranded sequencing adapter is linked to one end of the target double-stranded polynucleotide, and the other end of the target double-stranded polynucleotide is provided in a hairpin-shaped structure, for example formed by linking the hairpin-shaped adapter, such that the sequencing library is constructed. The constructed sequencing library may be preserved for long-term storage following conventional methods applicable to double-stranded polynucleotides. The constructed sequencing library consists of a strand A and a strand B, wherein the strand B is a strand to be sequenced and the strand A is a complementary strand of the strand to be sequenced. A 3' end of the strand A is capable of complementarily pairing with a primer strand C, a 5' end of the strand B is provided with a guide sequence (indicated by a black dotted line in the figure), a 3' end of the guide sequence is linked to an inhibitory segment (indicated by the bold black line in the figure) inhibiting the strand-displacement polymerization activity of a polymerase. The specific form of the inhibitory segment is not limited, as long as the inhibitory molecule is capable of inhibiting the strand-displacement polymerization activity of the polymerase. For example, the inhibitory segment may be a GC-rich motif with stronger binding ability, an artificially modified nucleotide (e.g., LNA, PNA, BNA), or other inhibitory molecules. Both the guide sequence and the inhibitory segment are positioned on the strand B. The whole sequencing process is as follows: (i) Mixing and incubating the sequencing library (strand A and strand B), the primers (strand C), and the polymerase with strand-displacement activity (represented by an ellipse in the figure), wherein the strand C is complementarily paired with the 3' end of the strand A, and the polymerase with strand-displacement activity binds to the target polynucleotide, thus forming the sequencing complex to be sequenced. (ii) Initiating a strand displacing polymerization reaction with the strand A serving as a template and the strand C serving as the primer, mediated by the polymerase with strand displacement activity, utilizing the necessary components in the sequencing buffer, such as pH buffer system, dNTP, Mg2+, etc. Wherein, the strand C is extended with the strand A serving as the template, as the strand B is displaced out, until the strand displacement polymerization activity of the polymerase is inhibited by the inhibitory segment. Since the 5' end of the strand C is attached with hydrophobic molecules such as cholesterol (represented as circles in the figure), the sequencing complex attached on the membrane material in the vicinity of the nanopore with the action of the hydrophobic molecules such as cholesterol (the membrane is represented as squares, and the center channel indicates the nanopore in the figure). With a sequencing voltage being applied, the guide sequence at the 5' end of the strand to be sequenced (the strand B) is captured by and passes through the nanopore under the electric field force (indicated by a downward arrow in the figure). (iii) Enabling the inhibitory segment to pass through the nanopore under the electric field force. As the strand B is displaced out, the strand C is extended in a stable speed with the strand A serving as the template, mediated by the polymerase with strand displacement activity. (iv) Enabling sequencing in a stable speed, as the strand B is displaced out and passes through the nanopore in a stable speed. (v) Melting the hairpin structure as the strand C extends to the end of the strand A, and further extending the strand C with the original hairpin structure sequence and the strand B serving as a template. As the strand C extends, the strand B exits from the nanopore in a direction inverse to the electric field, in a relatively stable speed (indicated by an upward arrow in the figure). (vi) Forming a new double strand as the strand C extends to the end of the strand B, when the strand B exits completely from the nanopore. The same Strand B is sequenced twice during the whole process.

In embodiments of the present disclosure, the polymerase having the strand displacement activity may be selected from DNA polymerase or RNA polymerase, such as a Bst DNA polymerase, a SD DNA polymerase, a phi29 DNA polymerase, a Bsu Large Fragment DNA polymerase, a Klenow Fragment DNA polymerase, a T3 RNA polymerase, a T7 RNA polymerase, a SP6 RNA polymerase, an *E.coli* RNA polymerase, or any combination thereof; or the polymerase is a modified polymerase having the strand displacement activity, which is a polymerase without strand displacement activity originally, such as a modified T4 DNA polymerase, a modified T7 DNA polymerase, a modified DNA polymerase I, or any combination thereof.

### Example 1: Screening for salt-resistant polymerases with strand displacement activity.

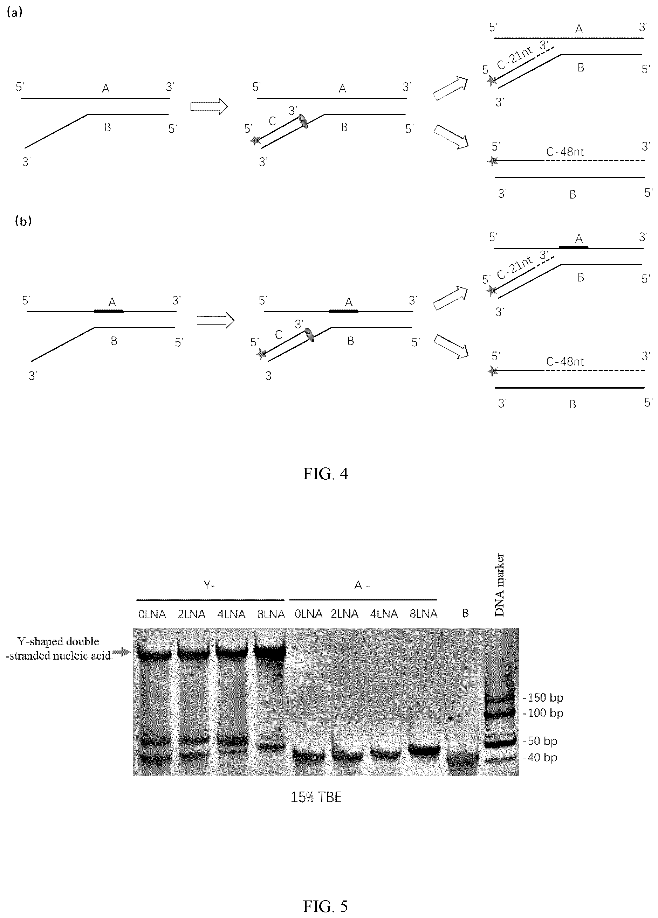

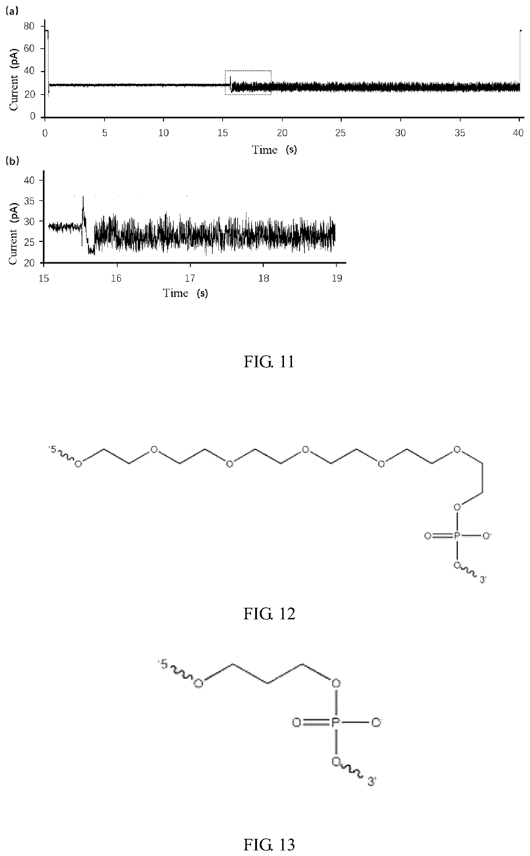

In FIG. 4, (a) showed a schematic diagram of the experiment of Example 1. Polynucleotides After annealing, SEQ.1 (strand A) and SEQ.2 (strand B) formed a Y-shaped double stranded nucleic acid structure, which was named as Y-0LNA; SEQ.3 (strand C) was 15 nt in length with a CY3 fluorescent modification (indicated by the star symbol) at the 5' end and was complementarily paired with the 3' end of SEQ.2 (strand B); the polymerase (indicated by oval symbols) binded to the nucleic acid complex. FIG. 13 showed the specific structure of iSpC3 therein. In the reaction buffer, the strand C is extended with the strand B serving as a template, mediated by the polymerase; if the polymerase lacked a polymerization activity, the strand C would not be extended and remained it length at 15 nt; if the polymerase lacked a strand displacement polymerization activity, the strand A would not be displaced out and the strand C would only be extended up to 21 nt in length; if the polymerase had a strand displacement polymerization activity, the strand A would be completely displaced out and the strand C would be extended up to 48 nt in length. The length of the strand C was detected by electrophoresis.
1. Annealing of Y-shaped double-stranded nucleic acid
1) SEQ.1, SEQ.2, and SEQ.3 were dissolved, respectively, in TE buffer (pH = 8) to obtain master mixs with a final concentration of 100 µM, according to the manufacturer's instructions.
2) SEQ.1 and SEQ.2 were annealed to the Y-shaped double-stranded nucleic acid, named Y-0LNA. The annealing process was that incubating at 95°C for 5 min, cooling with a rate of 0.1°C /s down to 25°C, and incubating again for 30 min. The annealing reaction system was shown in Table 1.

**Table 1 Annealing reaction system for Y-shaped double-stranded nucleic acid**

| **Reagents** | **Volume** |
|---|---|
| 100 µM SEQ.1 | 10 µL |
| 100 µM SEQ.2 | 10 µL |
| TE buffer (pH = 8) | 80 µL |
| **Total volume** | **100 µL** |

(3) Polynucleotide SEQ.1 as strand A was annealed with SEQ.2 as strand B. The polynucleotides and annealing products were subjected to 15% TBE native polyacrylamide gel electrophoresis at 200 V for 1 h. The electrophoresis results were shown in FIG. 5, lane 1 was the annealing product of SEQ.1+SEQ.2 (Y-0LNA) (with arrows indicating the Y-shaped double-stranded nucleic acid), lane 5 was SEQ.1, lane 9 was SEQ.2, and lane 10 wasthe DNA marker (Thermo Scientific, SM1313). The above results showed that SEQ.1 and SEQ.2 were able to form Y-shaped double-stranded nucleic acid as expected.
2. Screening of salt-resistant polymerases with strand displacement activity
(1) As shown in (a) in FIG. 4, the annealing products of SEQ.1 and SEQ.2 (strand A + strand B), Y-0LNA, SEQ.3 (strand C) with CY3 fluorescence modification at the 5' end, and polymerases with strand displacement activity were mixed with reaction buffer containing a high concentration of salt ions, and incubated for 1 hour at 30°C. The tested polymerases with strand displacement activity were BST+ (ArcticZymes Technologies, 71502-201); SD+ (ArcticZymes Technologies, 71501-201); Klenow Fragment (NEB, M0212S); Bsu Large Fragment (NEB, M0330S); phi29 (NEB, M0269S). The reaction buffers were: reaction buffer 1 (final concentration: 0.15 M KCl, 50 mM Tris-HCl, 10 mM MgCl2, 1 mM dNTP, pH=7.90); and reaction buffer 2 (final concentration: 0.30 M KCl, 50 mM Tris-HCl, 10 mM MgCl2, 1 mM dNTP, pH=8.10). The polymerase reaction system was shown in Table 2.

**Table 2 Polymerase reaction system**

| **Reagents** | **Volume** |
|---|---|
| 10 µM Y-0LNA | 1 µL |
| 10 µM SEQ.3 | 1 µL |
| polymerase | 0.2 µL |
| 2× reaction buffer | 5 µL |
| H₂O | 2.8 µL |
| **Total volume** | **10 µL** |

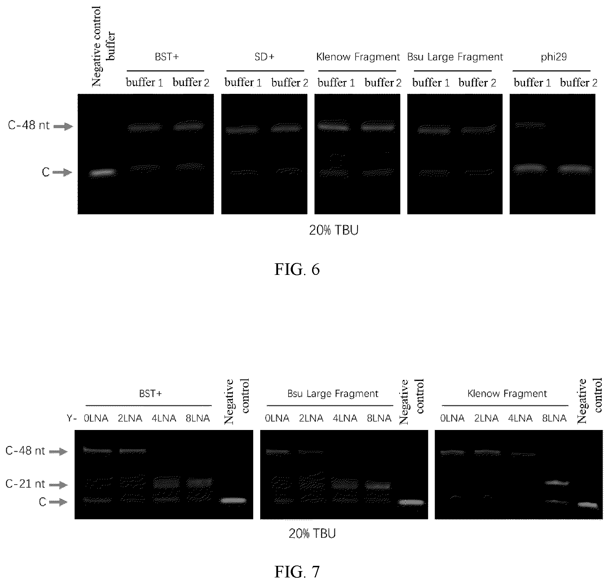

(2) 10 µL of reaction product was mixed with 10 µL of 2×sampling buffer (Invitrogen, AM8546G) and treated with 95°C for 10 min. The samples were subjected to 20% TBU denaturing polyacrylamide gel electrophoresis at 200 V for 1 h. CY3 fluorescence was detected after the electrophoresis. The electrophoresis results were shown in FIG. 6, lane 1 was SEQ.3 with CY3 fluorescence modification at the 5' end, as negative control (NC); and the products of strand displacement polymerization reactions at 0.15 M KCl and 0.30 M KCl by polymerase BST+ were shown in lanes 2-3, by polymerase SD+ were shown in lanes 4-5, by polymerase Klenow Fragment were shown in lanes 6-7, by polymerase Bsu Large Fragment were shown in lanes 8-9, by polymerase phi29 were shown in lanes 10-11. For the electrophoretic positions, strand C extended up to 48 nt was indicated by C-48 nt, and strand C not extended was indicated by C.
(3) The results shown in FIG. 6 indicated that: polymerase BST+, polymerase SD+, polymerase Klenow Fragment, and polymerase Bsu Large Fragment had strand displacement polymerization activity under the high salt conditions of 0.15 M KCl and 0.30 M KCl; polymerase phi29 had strand displacement polymerization activity under the high salt conditions of 0.15 M KCl; and polymerase phi29 had strand displacement polymerization activity under the condition of 0.15 M KCl and almost no strand displacement polymerization activity under the condition of 0.30 M KCl.

### Example 2: Detecting the inhibition for strand displacement polymerization activity of polymerase by modified nucleotide LNA in non-template strand.

In (b) of FIG. 4, a schematic diagram of the experiment of Example 2 was shown. As the strand A, polynucleotides SEQ.4, SEQ.5, and SEQ.6, which contained 2 LNAs, 4 LNAs, and 8 LNAs respectively (indicated by bold black line segments), were annealed with SEQ.2 (strand B) to obtain a Y-shaped double-stranded nucleic acid structure, which were named Y-2LNA, Y-4LNA, and Y-8LNA, respectively; and SEQ.3 (strand C) was 15 nt in length, and had a CY3 fluorescence modification at the 5' end (indicated by star symbol), and was complementary pairing with the 3' end of SEQ.2 (strand B); polymerase with strand displacement activity (indicated by ellipse symbol) bound to the nucleic acid complex. In the reaction buffer, the strand C is extended with the strand B serving as a template, mediated by the polymerase; if the polymerase lacked a polymerization activity, the strand C would not be extended and remained it length at 15 nt; if 2 LNAs, or 4 LNAs, or 8 LNAs contained in SEQ.4, or SEQ.5, or SEQ.6 were capable of inhibiting the strand-displacement polymerization activity of the polymerase, the strand A would not be displaced out and the strand C would only be extended up to 21 nt in length; if 2 LNAs, or 4 LNAs, or 8 LNAs contained in SEQ.4, or SEQ.5, or SEQ.6 were not capable of inhibiting the strand-displacement polymerization activity of the polymerase, the strand A would be completely displaced out and the strand C would be extended up to 48 nt in length. The length of the strand C was detected by electrophoresis.
1. Annealing of Y-shaped double-stranded nucleic acid
1) SEQ.1, SEQ.2, SEQ.3, SEQ.4, SEQ.5, and SEQ.6 were dissolved, respectively, in TE buffer (pH = 8) to obtain master mixes with a final concentration of 100 µM, according to the manufacturer's instructions.
(2) SEQ.1, SEQ.4, SEQ.5, and SEQ.6 were annealed with SEQ.2, respectively, to multiple Y-shaped double-stranded nucleic acid, and the annealing products were named Y-0LNA, Y-2LNA, Y-4LNA, and Y-8LNA, respectively; Y-0LNA was used as a control without LNA. The annealing process was that incubating at 95°C for 5 min, cooling with a rate of 0.1°C /s down to 25°C, and incubating again for 30 min. The annealing reaction system is shown in Table 3.

**Table 3 Annealing reaction system for Y-shaped double-stranded nucleic acid**

| **Reagents** | **Volume** |
|---|---|
| 100 µM SEQ.1/SEQ.4/SEQ.5/SEQ.6 | 10 µL |
| 100 µM SEQ.2 | 10 µL |
| TE buffer (pH = 8) | 80 µL |
| **Total volume** | **100 µL** |

(3) The polynucleotides and annealing products were subjected to 15% TBE native polyacrylamide gel electrophoresis at 200 V for 1 h. The electrophoretic results were shown in Fig. 5, lanes 1-4 were the annealed products of SEQ.1+ SEQ.2 (Y-0LNA), SEQ.4+ SEQ.2 (Y-2LNA), SEQ.5+ SEQ.2 (Y-4LNA), and SEQ.6+ SEQ.2 (Y-8LNA) (with arrows indicating the Y-shaped double-stranded nucleic acid), and lanes 5-9 were SEQ.1, SEQ.4, SEQ.5, SEQ.6, SEQ.2, respectively, and lane 10 was DNA marker (Thermo Scientific, SM1313). The above results showed that SEQ.1 and SEQ.2, SEQ.4 and SEQ.2, SEQ.5 and SEQ.2, and SEQ.6 and SEQ.2 were able to form Y-shaped double-stranded nucleic acid as expected.
2. Detecting the inhibition for strand displacement polymerization activity of polymerase by modified nucleotide LNA in non-template strand
1) As shown in (b) in Fig. 4, the annealing product Y-0LNA of SEQ.1 and SEQ.2 ( strand A + strand B) (as a control without LNA), or the annealing product Y-2LNA of SEQ.4 and SEQ.2 (strand A + strand B), or the annealing product Y-4LNA of SEQ.5 and SEQ.2 strand A + strand B), or the annealing product Y-8LNA of SEQ.6 and SEQ.2 (strand A + strand B), was mixed with SEQ.3 (strand C) with CY3 fluorescence modification at the 5' end, polymerase with strand displacement activity, and reaction buffer containing a high concentration of salt ions, and incubated for 1 hour at 30°C. The tested polymerases with strand displacement activity were BST+ (ArcticZymes Technologies, 71502-201); Klenow Fragment (NEB, M0212S); and Bsu Large Fragment (NEB, M0330S). The reaction buffers were: reaction buffer 2 (final concentration: 0.30 M KCl, 50 mM Tris-HCl, 10 mM MgCl2, 1 mM dNTP, pH=8.10). The polymerase reaction system was shown in Table 4.

**Table 4 Polymerase reaction system**

| **Reagents** | **Volume** |
|---|---|
| 10 µM Y-0LNA/ Y-2LNA/ Y-4LNA/ | 1 µL |
| Y-8LNA | |
| 10 µM SEQ.3 | 1 µL |
| polymerase | 0.2 µL |
| 2× reaction buffer | 5 µL |
| H₂O | 2.8 µL |
| **Total volume** | **10 µL** |

(2) 10 µL of reaction product of the step (1) was mixed with 10 µL of 2×sampling buffer (Invitrogen, AM8546G) and treated with 95°C for 10 min. The samples were subjected to 20% TBU denaturing polyacrylamide gel electrophoresis at 200 V for 1 h. CY3 fluorescence was detected after the electrophoresis. The electrophoresis results were shown in FIG. 7, the products of strand displacement polymerization reactions at 0.30 M KCl by polymerase BST+ were shown in lanes 1-4, by polymerase were shown in Bsu Large Fragment lanes 6-9, by polymerase Klenow Fragment were shown in lanes 11-14; lanes 5, 10, and 15 were SEQ.3 with CY3 fluorescence modification at the 5' end, as negative control (NC). For the electrophoretic positions, strand C extended up to 48 nt was indicated by C-48 nt, strand C extended up to 21 nt was indicated by C-21 nt, and strand C not extended was indicated by C.

The results shown in FIG. 7 demonstrated that, under the high salt condition of 0.30 M KCl, four or eight LNAs in the non-template strand were able to well inhibit the strand displacement polymerization activities of polymerase BST+, polymerase Klenow Fragment, and polymerase Bsu Large Fragment; and two LNAs in the non-template strand were able to partially inhibit the strand displacement polymerization activities of polymerase BST+, polymerase Klenow Fragment, polymerase Bsu Large Fragment.

### Example 3: Construction of sequencing library

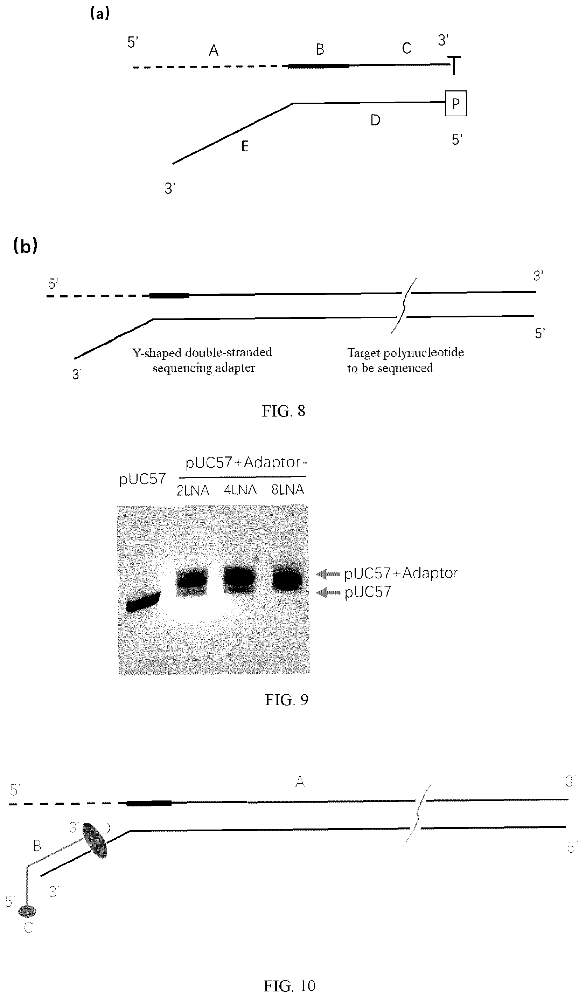

FIG. 8 showed a schematic diagram of the sequencing library construction in Example 3. (a) showed a schematic diagram of a sequencing adapter.

The top strand (SEQ.7, SEQ.8, SEQ.9) consisted of parts A, B, and C. Part A was a guide sequence, consisting of 30 iSpC3s (indicated by a black dashed line in the figure); part B was a inhibitory segment, which was a segment capable of inhibiting polymerase strand displacement polymerization activity, consisting of two, four, and eight LNA-modified nucleic acids, respectively (indicated by a thickened black line segment in the figure); and part C was a DNA capable of complementarily pairing to the bottom strand, having a protruding T at the 3' end. The bottom strand (SEQ.10) had a modification of a phosphate group at the 5' end and consisted of parts D and E. Part D was the sequence capable of complementarily pairing to parts B and C of the top strand, part E was the sequence capable of complementarily pairing to the primer (SEQ.11). FIG. 12 showed a specific structure of iSp18 therein. In a ratio of 1:1, the top and bottom strands were annealed into the Y-shaped double-stranded sequencing adaptors. Respective adapters were indicated as follows: the adapter of annealing SEQ.7 and SEQ.10 was indicated as Adaptor-2LNA, the adapter of annealing SEQ.8 and SEQ.10 was indicated as Adaptor-4LNAm and the adapter of annealing SEQ.9 and SEQ.10 was indicated as Adaptor-8LNA. The (b) showed a schematic diagram of the sequencing library. The target polynucleotide to be sequenced (pUC57 plasmid digested linearized double-stranded DNA) was linked to the Y-shaped double-stranded sequencing adaptor after the end repair and A-tailing, then after a magnetic bead purification, the library to be sequenced was obtained.
1. Annealing of Y-shaped double-stranded sequencing adapter
   1) SEQ.7, SEQ.8, SEQ.9, and SEQ.10 were dissolved, respectively, in TE buffer (pH = 8) to obtain master mixes with a final concentration of 100 µM, according to the manufacturer's instructions.
   2) As shown in FIG. 8,

SEQ.7, SEQ.8, and SEQ.9 were annealed with SEQ.10, respectively, to multiple Y-shaped double-stranded sequencing adapters, and the annealing products obtained were named Adaptor-2LNA, Adaptor-4LNA, and Adaptor-8LNA, respectively. The annealing process was that incubating at 95°C for 5 min, cooling with a rate of 0.1°C /s down to 25°C, and incubating again for 30 min. The annealing reaction system is shown in Table 5.

**Table 5 Annealing reaction system for Y-shaped double-stranded sequencing adapter**

| **Reagents** | **Volume** |
|---|---|
| 100 µM SEQ.7/SEQ.8/SEQ.9 | 10 µL |
| 100 µM SEQ.10 | 10 µL |
| TE buffer (pH = 8) | 80 µL |
| **Total volume** | **100 µL** |

2. Y-shaped double-stranded sequencing adapters and DNA to be tested were linked to form the sequencing library
1) The pUC57 plasmid (SEQ.12) was digested with restriction endonucleases EcoR I-HF (NEB, R3101) and Hind III-HF (NEB, R3104) according to the manufacturer's instructions, and the reaction condition was incubating at 37°C for 60 min. The enzyme digestion reaction system was shown in Table 6.

**Table 6 Enzyme digestion reaction system**

| **Reagents** | **Mass/volume** |
|---|---|
| pUC57 plasmid | 5 µg |
| rCutSmart^{™} Buffer | 5 µL |
| EcoR I-HF | 1 µL |
| Hind III-HF | 1 µL |
| H₂O | Make up to 50 µL |
| **Total volume** | **50 µL** |

2) Purification of the digest products was performed with AMPure XP beads (Beckman Coulter, A63882) according to the manufacturer's instructions and the product concentration was determined using the Qubit dsDNA HS kit (Thermofisher, Q32854).
3) The end repair and A-tailing of the purified digest products were performed with NEBNext FFPE DNA Repair Mix (NEB, M6630) and NEBNext Ultra II End repair / dA-tailing Module (NEB, E7546) according to the manufacturer's instructions. The reaction conditions were incubating at 20°C for 5 min, and then incubating at 65°C for 5 min. The reaction system for end repair and A-tailing was shown in Table 7.

**Table 7 End-repair and A-tailing reaction systems**

| **Reagents** | **Mass/volume** |
|---|---|
| purified digest products | 1 µg |
| NEBNext FFPE DNA Repair Buffer | 3.5 µL |
| NEBNext FFPE DNA Repair Mix | 2 µL |
| Ultra II End-prep reaction buffer | 3.5 µL |
| Ultra II End-prep enzyme mix | 3 µL |
| H₂O | Make up to 60 µL |
| **Total volume** | **60 µL** |

4) The products after End repair and A-tailing were purified with AMPure XP beads (Beckman Coulter, A63882) according to the manufacturer's instructions, and product concentrations were determined using the Qubit dsDNA HS kit (Thermofisher, Q32854).
5) The purified products were subjected to a ligation reaction using NEBNext Quick Ligation Module (NEB, E6056) according to the manufacturer's instructions, the reaction condition was incubating at 25°C for 10 min. The ligation reaction system was shown in Table 8.

**Table 8 Ligation Reaction Systems**

| **Reagents** | **Volume** |
|---|---|
| Purified products after End repair and A-tailing | 60 µL |
| 10 µM Adaptor-2LNA/ Adaptor-4LNA/ Adaptor-8LNA | 3 µL |
| NEBNext Quick Ligation Reaction Buffer | 25 µL |
| Quick T4 DNA Ligase | 10 µL |
| H₂O | Make up to 100 µL |
| **Total volume** | **100 µL** |

6) The adapter ligated products were purified with AMPure XP beads (Beckman Coulter, A63882) according to the manufacturer's instructions, and the product concentrations were determined using the Qubit dsDNA HS kit (Thermofisher, Q32854). The sequencing library as shown in FIG. 8 was obtained.
7) The sequencing library was subjected to 6% TBE n native polyacrylamide gel electrophoresis at 200 V for 2 h. The results of electrophoresis were shown in FIG. 9. Lane 1 was the target polynucleotide to be tested before ligation (the pUC57 plasmid digested linearized double-stranded DNA), and lanes 2-4 were the purified products of sequencing adapters containing 2, 4, and 8 LNA-modified nucleic acids ligated to the target polynucleotide to be tested, respectively, named as pUC57+Adaptor-2LNA, pUC57+Adaptor-4LNA, and pUC57+Adaptor-8LNA. For the electrophoretic positions, the adapter-ligated sequencing libraries were indicated by "pUC57+Adaptor", and the target polynucleotides being not adapter-ligated were indicated by "pUC57". The results shown in FIG. 9 indicated that the three sequencing libraries pUC57+Adaptor-2LNA, pUC57+Adaptor-4LNA and pUC57+Adaptor-8LNA were successfully constructed.

### Example 4: Performing nanopore sequencing.

1) The sequencing library pUC57+Adaptor-4LNA was incubated with primer (SEQ.11), polymerase BST+ with strand displacement activity and the reaction buffer at 30°C for 1 h, to form the sequencing complex shown in FIG. 10. In FIG. 10, the sequencing library labeled A was complementarily paired with the primer (with a cholesterol modification labeled C at the 5' end) labeled B, and in the meanwhile, bound with the polymerase with strand displacement activity (labeled D) by incubation, such that forming the sequencing complex. The final concentrations of the reaction buffer were 37.5 mM KCl, 12.5 mM Tris-HCl, 2.5 mM MgCl2 , pH=8.10. The incubation system for sequencing complex was shown in Table 9.

**Table 9 Incubation reaction system for sequencing complex**

| **Reagents** | **Volume** |
|---|---|
| 100 ng/µL sequencing library | 5 µL |
| 1 µM primer | 5 µL |
| BST+ | 5 µL |
| reaction buffer | 5 µL |
| **Total volume** | **20 µL** |

2) A single-channel nanopore detection system based on membrane clamp and signal amplifier was built, and a single pore protein was embedded, according to the literature (Ji Z, Guo P. Channel from bacterial virus T7 DNA packaging motor for the differentiation of peptides composed of a mixture of acidic and basic amino acids. Biomaterials. 2019 May 21;214:119222) disclosed method. The sequencing complex after incubation was add into this single-channel system, to observe and obtain the change of current signal under 100 mV, wherein the sequencing buffer were 0.30 M KCl, 50 mM Tris-HCl, 10 mM MgCl2, 0.5 mM dNTP, pH=8.10, and labeled as the first group.
The sequencing results were shown in FIG. 11. Sequencing signals of pUC57+Adaptor-4LNA sequencing library were captured, and clear current amplitude changes of sequencing signals were obtained. In FIG. 11, (a) showed the current signal change image after the sequencing library was captured by the nanopore. The (b) showed a magnified view of the signal in the box in (a).
3) The exact same experimental procedure was performed for the second group of tests, except for the different sequencing buffer used. The sequencing buffer used in the second group was 0.30 M KCl, 50 mM Tris-HCl, 10 mM MgCl2, pH=8.10, and without dNTP. Then no sequencing signal was captured. The sequencing buffer and sequencing results were shown in Table 10.

**Table 10 Sequencing buffer and sequencing results**

| **Groups** | **Sequencing Buffer** | **Sequencing results** |
|---|---|---|
| Group I | 0.30 M KCl, 50 mM Tris-HCl, 10 mM MgCl2, 0.5 mM dNTP (pH=8.10) | A clear image of the current amplitude change of the sequencing signal was obtained |
| Group II | 0.30 M KCl, 50 mM Tris-HCl, 10 mM MgCl2, (pH=8.10) | No sequencing signal was captured |

The results shown in Table 10 indicated that sequencing can be performed using the polymerase BST+ with strand displacement activity and pUC57+Adaptor-4LNA sequencing libraries in the presence of dNTP in the sequencing buffer. It was demonstrated that the sequencing protocol of the present disclosure is feasible.

### Sequencing List

SEQ.1: 
5’

XXXXXXXXXXXXXXXXXXXXXXXXXXXXXXGGTTGTTTCTGTTGGTGCTGATATTG

C 3’ (X=iSpC3)
SEQ.2:
   5' GCAATATCAGCACCAACAGAAACAACCTCGGGTCGTAAGAATTCTATT 3'
SEQ.3:
   5' CY3-AATAGAATTCTTACG 3'
SEQ.4: 
5’

XXXXXXXXXXXXXXXXXXXXXXXXXXXXXX(LNA_G)(LNA_G)TTGTTTCTGTTGGT

GCTGATATTGC 3’ (X=iSpC3)
SEQ.5: 
5’

XXXXXXXXXXXXXXXXXXXXXXXXXXXXXX(LNA_G)(LNA_G)(LNA_T)(LNA_T)GTT

TCTGTTGGTGCTGATATTGC 3’ (X=iSpC3)
SEQ.6: 5' 
XXXXXXXXXXXXXXXXXXXXXXXXXXXXXX(LNA_G)(LNA_G)(LNA_T)(LNA_T)(LN
A_G)(LNA_T)(LNA_T)(LNA_T)CTGTTGGTGCTGATATTGC 3’ (X=iSpC3)
SEQ.7: 
5’
XXXXXXXXXXXXXXXXXXXXXXXXXXXXXX(LNA_G)(LNA_G)TTGTTTCTGTTGGT
GCTGATATTGCT 3’ (X=iSpC3)
SEQ.8: 
5’

XXXXXXXXXXXXXXXXXXXXXXXXXXXXX(LNA_G)(LNA_G)(LNA_T)(LNA_T)GTT

TCTGTTGGTGCTGATATTGCT 3’ (X=iSpC3)
SEQ.9: 
5’

XXXXXXXXXXXXXXXXXXXXXXXXXXXXXX(LNA_G)(LNA_G)(LNA_T)(LNA_T)(LN

A_G)(LNA_T)(LNA_T)(LNA_T)CTGTTGGTGCTGATATTGCT 3’ (X=iSpC3)
SEQ.10:
   5' Phosphorylation-GCAATATCAGCACCAACAGAAACAACCTTTGAGGCGAGCGGTCAA 3'
SEQ.11:
   5' Cholesterol-YTTGACCGCTCGC 3' (Y=iSp18)
SEQ.12:

5’
TCGCGCGTTTCGGTGATGACGGTGAAAACCTCTGACACATGCAGCTCCCGGAGACGG
TCACAGCTTGTCTGTAAGCGGATGCCGGGAGCAGACAAGCCCGTCAGGGCGCGTCAG
CGGGTGTTGGCGGGTGTCGGGGCTGGCTTAACTATGCGGCATCAGAGCAGATTGTACT
GAGAGTGCACCATATGCGGTGTGAAATACCGCACAGATGCGTAAGGAGAAAATACCG
CATCAGGCGCCATTCGCCATTCAGGCTGCGCAACTGTTGGGAAGGGCGATCGGTGCG
GGCCTCTTCGCTATTACGCCAGCTGGCGAAAGGGGGATGTGCTGCAAGGCGATTAAGT
TGGGTAACGCCAGGGTTTTCCCAGTCACGACGTTGTAAAACGACGGCCAGTGAATTC
GAGCTCGGTACCTCGCGAATGCATCTAGATATCGGATCCCGGGCCCGTCGACTGCAGA
GGCCTGCATGCAAGCTTGGCGTAATCATGGTCATAGCTGTTTCCTGTGTGAAATTGTTA
TCCGCTCACAATTCCACACAACATACGAGCCGGAAGCATAAAGTGTAAAGCCTGGGG
TGCCTAATGAGTGAGCTAACTCACATTAATTGCGTTGCGCTCACTGCCCGCTTTCCAGT

CGGGAAACCTGTCGTGCCAGCTGCATTAATGAATCGGCCAACGCGCGGGGAGAGGCG
GTTTGCGTATTGGGCGCTCTTCCGCTTCCTCGCTCACTGACTCGCTGCGCTCGGTCGTT
CGGCTGCGGCGAGCGGTATCAGCTCACTCAAAGGCGGTAATACGGTTATCCACAGAAT
CAGGGGATAACGCAGGAAAGAACATGTGAGCAAAAGGCCAGCAAAAGGCCAGGAAC
CGTAAAAAGGCCGCGTTGCTGGCGTTTTTCCATAGGCTCCGCCCCCCTGACGAGCATC
ACAAAAATCGACGCTCAAGTCAGAGGTGGCGAAACCCGACAGGACTATAAAGATACC
AGGCGTTTCCCCCTGGAAGCTCCCTCGTGCGCTCTCCTGTTCCGACCCTGCCGCTTAC
CGGATACCTGTCCGCCTTTCTCCCTTCGGGAAGCGTGGCGCTTTCTCATAGCTCACGCT
GTAGGTATCTCAGTTCGGTGTAGGTCGTTCGCTCCAAGCTGGGCTGTGTGCACGAACC
CCCCGTTCAGCCCGACCGCTGCGCCTTATCCGGTAACTATCGTCTTGAGTCCAACCCG
GTAAGACACGACTTATCGCCACTGGCAGCAGCCACTGGTAACAGGATTAGCAGAGCG
AGGTATGTAGGCGGTGCTACAGAGTTCTTGAAGTGGTGGCCTAACTACGGCTACACTA
GAAGAACAGTATTTGGTATCTGCGCTCTGCTGAAGCCAGTTACCTTCGGAAAAAGAGT
TGGTAGCTCTTGATCCGGCAAACAAACCACCGCTGGTAGCGGTGGTTTTTTTGTTTGC
AAGCAGCAGATTACGCGCAGAAAAAAAGGATCTCAAGAAGATCCTTTGATCTTTTCTA
CGGGGTCTGACGCTCAGTGGAACGAAAACTCACGTTAAGGGATTTTGGTCATGAGATT
ATCAAAAAGGATCTTCACCTAGATCCTTTTAAATTAAAAATGAAGTTTTAAATCAATCT
AAAGTATATATGAGTAAACTTGGTCTGACAGTTACCAATGCTTAATCAGTGAGGCACCT
ATCTCAGCGATCTGTCTATTTCGTTCATCCATAGTTGCCTGACTCCCCGTCGTGTAGATA
ACTACGATACGGGAGGGCTTACCATCTGGCCCCAGTGCTGCAATGATACCGCGAGACC
CACGCTCACCGGCTCCAGATTTATCAGCAATAAACCAGCCAGCCGGAAGGGCCGAGC
GCAGAAGTGGTCCTGCAACTTTATCCGCCTCCATCCAGTCTATTAATTGTTGCCGGGAA
GCTAGAGTAAGTAGTTCGCCAGTTAATAGTTTGCGCAACGTTGTTGCCATTGCTACAG
GCATCGTGGTGTCACGCTCGTCGTTTGGTATGGCTTCATTCAGCTCCGGTTCCCAACGA
TCAAGGCGAGTTACATGATCCCCCATGTTGTGCAAAAAAGCGGTTAGCTCCTTCGGTC
CTCCGATCGTTGTCAGAAGTAAGTTGGCCGCAGTGTTATCACTCATGGTTATGGCAGC
ACTGCATAATTCTCTTACTGTCATGCCATCCGTAAGATGCTTTTCTGTGACTGGTGAGT
ACTCAACCAAGTCATTCTGAGAATAGTGTATGCGGCGACCGAGTTGCTCTTGCCCGGC
GTCAATACGGGATAATACCGCGCCACATAGCAGAACTTTAAAAGTGCTCATCATTGGA
AAACGTTCTTCGGGGCGAAAACTCTCAAGGATCTTACCGCTGTTGAGATCCAGTTCGA
TGTAACCCACTCGTGCACCCAACTGATCTTCAGCATCTTTTACTTTCACCAGCGTTTCT
GGGTGAGCAAAAACAGGAAGGCAAAATGCCGCAAAAAAGGGAATAAGGGCGACAC

GGAAATGTTGAATACTCATACTCTTCCTTTTTCAATATTATTGAAGCATTTATCAGGGTT
ATTGTCTCATGAGCGGATACATATTTGAATGTATTTAGAAAAATAAACAAATAGGGGTT
CCGCGCACATTTCCCCGAAAAGTGCCACCTGACGTCTAAGAAACCATTATTATCATGA
CATTAACCTATAAAAATAGGCGTATCACGAGGCCCTTTCGTC 3'

Although embodiments of the present disclosure have been shown and described, it would be appreciated by those skilled in the art that the above embodiments are only explanatory, and cannot be construed to limit the present disclosure, and changes, alternatives, and modifications can be made in the embodiments in the scope of the present disclosure.

## Claims

1. A method for nanopore sequencing, comprising:
i) providing a sequencing library, wherein the sequencing library comprises a target double stranded polynucleotide, the target double stranded polynucleotide comprises a first strand and a second strand, a 5' end of the first strand and a 3' end of the second strand form an first unpaired single-stranded region, the first unpaired single-stranded region is provided with a guide sequence at the 5' end of the first strand, the first unpaired single-stranded region is provided with a second primer pairing region at the 3' end of the second strand;
ii) incubating the sequencing library with a first primer to obtain a sequencing complex, wherein the first primer binds to the second primer pairing region based on a base complementary pairing principle, the first primer has a free 3' end and has a 5' end capable of attaching the complex to a membrane with a nanopore embedded;
iii) extending the first primer with the second strand serving as a template mediated by a first polymerase having a strand displacement activity, wherein, under an electric field force, the guide sequence is captured by the nanopore embedded in the membrane and passes through the nanopore, and the first strand of the target double stranded polynucleotide is displaced out and passes through the nanopore as the first primer extends; and
iv) detecting the electrical signal changes generated as the first strand passes through the nanopore during extension, to determine sequence information for the first strand of the target double stranded polynucleotide.

2. The method of claim 1, wherein in i), the 3' end of the first strand and the 5' end of the second strand form a paired region under completely complementarily pairing.

3. The method of claim 1, wherein in i), in the target double stranded polynucleotide, a 3' end of the first strand and a 5' end of the second strand form a second unpaired single-stranded region, the second unpaired single-stranded region is provided with a first primer pairing region at the 3' end of the first strand.

4. The method of claim 3, further comprising:
in ii), incubating the sequencing library with the first primer, a second primer to obtain the sequencing complex, wherein the first primer binds to the second primer pairing region based on the complementary base pairing rule, the second primer binds to the first primer pairing region based on the base complementary pairing principle, and the 5' end(s) of the first primer and/or the second primer is(are) capable of attaching the complex to the membrane with the nanopore embedded.

5. The method of claim 4, further comprising:
v) extending the second primer with the first strand serving as a template mediated by a second polymerase such that the first strand exits the nanopore embedded in the membrane in a direction inverse to the electric field, and detecting the electrical signal changes generated as the first strand exits the nanopore, to determine the sequence information for the first strand of the target double stranded polynucleotide again.

6. The method of claim 4 or 5, wherein the second strand is provided with a guide sequence at the 5' end.

7. The method of claim 1, wherein in i), in the target double stranded polynucleotide, a 3' end of the first strand and a 5' end of the second strand are connected, such that the target double stranded polynucleotide is provided with a hairpin structure at the connection.

8. The method of claim 7, further comprising:
v) continuing extending with the first strand serving as a template mediated by the first polymerase after extending to the hairpin structure, such that the first strand exits the nanopore embedded in the membrane in a direction inverse to the electric field, and detecting the electrical signal changes generated as the first strand exits the nanopore, to determine the sequence information for the first strand of the target double stranded polynucleotide again.

9. The method of any one of claims 1 to 8, wherein the target double stranded polynucleotide is prepared by linking a Y-adapter to a double stranded polynucleotide, two strands of the Y-adapter comprise the guide sequence and a primer pairing region respectively, such that the target double stranded polynucleotide, obtained by linking the Y-adapter to the double stranded polynucleotide, is provided with the guide sequence at the 5' end of the first strand and the second primer pairing region at the 3' end of the second strand.

10. The method of any one of claims 1 to 8, wherein the primer is less than 80, 70, 60 or 50 nucleotides in length.

11. The method of any one of claims 1 to 8, wherein the 5' end of the primer is attached with a hydrophobic molecule.

12. The method of claim 11, wherein the hydrophobic molecule is selected from any one or more of the following: a lipid, fatty acid, a sterol, a carbon nanotube, a peptide, a protein and/or an amino acid.

13. The method of any one of claims 1 to 8, wherein the guide sequence comprises a polynucleotide or a plurality of iSpC3s.

14. The method of any one of claims 1 to 8, wherein the guide sequence is 10-50 nucleotides or 10-50 iSpC3s in length.

15. The method of any one of claims 1 to 8, wherein a 3' end of the guide sequence is linked to an inhibitory segment that inhibits the strand displacement activity of the polymerase.

16. The method of claim 15, wherein the inhibitory segment is a GC-rich motif or an artificially modified nucleotide.

17. The method of claim 16, wherein the artificially modified nucleotide is a locked nucleic acid (LNA), a peptide nucleic acid (PNA), a bridged nucleic acid (BNA), or any combination thereof.

18. The method of claim 17, wherein the artificially modified nucleotide is the LNA, whose number is 2-10, preferably 4-8.

19. The method of any one of claims 1 to 8, wherein the polymerase having the strand displacement activity is a DNA polymerase or an RNA polymerase.

20. The method of claim 19, wherein the polymerase having the strand displacement activity is a salt-tolerant DNA polymerase or salt- tolerant RNA polymerase.

21. The method of claim 19 or 20, wherein the polymerase having the strand displacement activity is: a Bst DNA polymerase, a SD DNA polymerase, a phi29 DNA polymerase, a Bsu Large Fragment DNA polymerase, a Klenow Fragment DNA polymerase, a T3 RNA polymerase, a T7 RNA polymerase, a SP6 RNA polymerase, an *E.coli* RNA polymerase, or any combination thereof.

22. The method of claim 19 or 20, wherein the polymerase is a modified polymerase having the strand displacement activity, which is a polymerase without strand displacement activity originally.

23. The method of claim 22, wherein the modified polymerase is a modified T4 DNA polymerase, a modified T7 DNA polymerase, a modified DNA polymerase I, or any combination thereof, having strand displacement activity.

24. The method of any one of claims 1 to 8, wherein the nanopore is a transmembrane protein pore or a solid-state pore.

25. The method of claim 24, wherein the transmembrane protein pore is selected from hemolysin, MspA, MspB, MspC, MspD, FraC, ClyA, PA63, CsgG, CsgD, XcpQ, SP1, phi29 connector protein, InvG, GspD, or any combination thereof.

26. The method of any one of claims 1 to 8, wherein the nanopore is further attached with an additional polypeptide selected from a tag, an enzyme cleavage site, a signaling peptide or a guide peptide, a detectable marker or any combination thereof.

27. The method of any one of claims 1 to 8, wherein the membrane is an amphiphilic membrane, a high-molecular polymer membrane or any combination thereof.

28. The method of claim 27, wherein the membrane is a phospholipid bilayer, a diblock copolymer or a triblock copolymer.

29. The method of any one of claims 1 to 8, wherein the method is carried out in a reaction buffer or sequencing buffer as follows: a dihydrogen phosphate-hydrogen phosphate buffer system, a carbonic acid-sodium bicarbonate buffer system, a Tris-HCl buffer system, a HEPES buffer system, a MOPS buffer system or any combination thereof.

30. The method of claim 29, wherein the reaction buffer or sequencing buffer contains an additive or an auxiliary reagent that enhances a polymerase extension reaction.

31. The method of claim 30, wherein the additive or the auxiliary reagent is selected from dimethyl sulfoxide, glycerol, formamide, bovine serum albumin, ammonium sulfate, polyethylene glycol, gelatin, non-ionic detergent, N,N,N-trimethylglycine, single-stranded nucleic acid-binding protein or any combination thereof.

32. A kit, comprising:
a Y-adapter for linking to a double stranded polynucleotide, wherein two strands of the Y-adapter comprise a guide sequence and a primer pairing region respectively, such that a target double stranded polynucleotide, obtained by linking the Y-adapter to the double stranded polynucleotide, is provided with the guide sequence at the 5' end of a first strand and the primer pairing region at the 3' end of a second strand;
a nanopore;
a membrane;
one or more primer, wherein the primer is complementary to the primer pairing region, and the primer has an attaching segment at the 5' end for attaching to the membrane;
one or more polymerase having a strand displacement activity;
a buffer system for a polymerization reaction.

33. The kit of claim 32, wherein the nanopore is embedded in the membrane.

34. The kit of claim 32 or 33, wherein a 3' end of the guide sequence is attached with an inhibitory segment that inhibits the strand displacement activity of the polymerase, and the inhibitory segment is a GC-rich motif or an artificially modified nucleotide.

35. The kit of claim 34, wherein the artificially modified nucleotide is LNA, PNA, BNA, or any combination thereof; preferably the artificially modified nucleotide is 2-10 LNAs, more preferably the artificially modified nucleotide is 4-8 LNAs .

36. The kit of claim 32 or 33, wherein the primer is less than 80, 70, 60 or 50 nucleotides in length, and a 5' end of the primer is attached with a hydrophobic molecule, and the hydrophobic molecule is selected from any one or more of the following: a lipid, fatty acid, a sterol, a carbon nanotube, a peptide, a protein and/or an amino acid.

37. The kit of claim 32 or 33, wherein the guide sequence comprises a polynucleotide or a plurality of iSpC3s, and the guide sequence is 10-50 nucleotides or 10-50 iSpC3 in length.

38. The kit of claim 32 or 33, wherein the polymerase is a Bst DNA polymerase, a SD DNA polymerase, a phi29 DNA polymerase, a Bsu Large Fragment DNA polymerase, a Klenow Fragment DNA polymerase, a T3 RNA polymerase, a T7 RNA polymerase, a SP6 RNA polymerase, an *E.coli* RNA polymerase, or any combination thereof; or the polymerase is a modified T4 DNA polymerase, a modified T7 DNA polymerase, a modified DNA polymerase I, or any combination thereof, having the strand displacement activity.

39. The kit of claim 32 or 33, wherein the nanopore is a transmembrane protein pore or a solid-state pore.

40. The kit of claim 39, wherein the transmembrane protein pore is selected from hemolysin, MspA, MspB, MspC, MspD, FraC, ClyA, PA63, CsgG, CsgD, XcpQ, SP1, phi29 connector protein, InvG, GspD, or any combination thereof.

41. The kit of claim 32 or 33, wherein the membrane is an amphiphilic membrane, a high-molecular polymer membrane or any combination thereof.

42. The kit of claim 41, wherein the membrane is a phospholipid bilayer, a diblock copolymer or a triblock copolymer.

43. The kit of claim 32 or 33, wherein the buffer system for the polymerization reaction is a dihydrogen phosphate-hydrogen phosphate buffer system, a carbonic acid-sodium bicarbonate buffer system, a Tris-HCl buffer system, a HEPES buffer system, a MOPS buffer system or any combination thereof.

44. The kit of claim 32 or 33, wherein the buffer system for the polymerization reaction contains an additive or an auxiliary reagent that enhances a polymerase extension reaction, and the additive or the auxiliary reagent is selected from the group consisting of dimethyl sulfoxide, glycerol, formamide, bovine serum albumin, ammonium sulfate, polyethylene glycol, gelatin, non-ionic detergent, N,N,N-trimethylglycine, single-stranded nucleic acid-binding protein or any combination thereof.

45. Use of the kit according to any one of claims 32 to 44 in high throughput sequencing.

46. The use of claim 45, wherein the high throughput sequencing is nanopore sequencing.
